# EUROPEAN PATENT APPLICATION

(11) **EP 2 392 380 A1**
(43) Date of publication of application: **07.12.2011**
(21) Application number: 11168990.7
(22) Date of filing: 07.06.2011
(51) Int. Cl.: A61M 39/22

(54) **Transfusion control tap**

(30) Priority: 07.06.2010 GB 1009418
(71) Applicant: Thies, Karl-Christian, Bromsgrove B61 8DW (GB)
(72) Inventor: Thies, Karl-Christian, Bromsgrove B61 8DW (GB)
(74) Representative: Gee, Steven William

(57) **Abstract**

This invention relates to a transfusion control tap (10), and in particular to a tap used in the transfusion supply line for a baby or small child. The transfusion control tap (10) has three flow lines (14, 16, 20) and a rotatable control member (26), the rotatable control member having lumens (30, 32) which can be aligned with respective flow lines to interconnect those flow lines. According to the invention the control member has only two lumens whereby only two of the flow lines can be interconnected at a time, and importantly in which a chosen two of the flow lines cannot be interconnected. The invention therefore avoids the possibility that a blood supply bag can be inadvertently connected directly to the patient.

## Description

### FIELD OF THE INVENTION

This invention relates to a transfusion control tap, and in particular to a tap used in the transfusion supply line for a baby or small child. The invention is expected to find its greatest utility in the transfusion of blood during emergency medical treatments, and the following description will therefore relate primarily to such applications. The invention is not, however, limited to such applications, and can be used for the transfusion of other fluids, and in non-emergency situations.

In addition, the tap can be of use in all situations where fluids, body fluids, medications, blood products, contrast media or gases are administered or withdrawn and where separation of the circuits is intended. Potential fields of use include haemodialysis, haemofiltration, extracorporeal membrane oxygenation and extracorporeal circulation (extracorporeal life support systems), transcutaneous catheter or tube insertion, interventional radiology and artificial ventilation.

### BACKGROUND TO THE INVENTION

Rapid intravenous administration of fluids or blood products is often necessary during surgical procedures or emergency medical treatments. Blood products or intravenous fluids (IV fluids) will typically be provided in a flexible bag. The bag will be suspended above the patient and the fluid provided to the patient by way of a supply line (intravenous giving set) and an intravascular catheter, the blood flowing to the patient under the force of gravity. It is also known to provide a pressurised blood flow by locating the blood bag into a vessel which can be filled with a gas or liquid at a controlled rate so as to compress the bag and force the blood through the supply line and catheter. Most blood product bags have a volume of 250-350 ml. Intravenous fluids are usually provided in bags of 500 ml. In the following text the term "blood" will be used for all types of blood products and IV fluids. The term "transfusion" will be used for all types of intravascular administration of blood products and fluids.

A flow regulator will typically be fitted to the supply line so that the flow rate of blood to the patient can be regulated. In addition, one or more control taps are used in the supply line, the control tap(s) allowing the flow of blood to be stopped and started as required, and also to permit the introduction of medicants into the blood. The known control taps therefore have three flow lines, arranged in a "T" formation. The control tap will typically be installed with the blood supply line and the patient's catheter line connected to the flow lines providing the top bar of the "T", with the third flow line (providing the leg of the "T") being used for the supply of medicants.

The control tap can be moved to a fully open position in which all of the flow lines are open, in which case blood can flow from the bag to the patient, and medicant can be introduced into the flowing blood. The control tap can be moved to a first alternative position in which only the flow lines connected to the blood supply line and the catheter line respectively are open, so that blood can flow to the patient. The control tap can be moved to a second alternative position in which only the flow lines connected to the medicant supply and the catheter line are open, so that blood does not flow to the patient but a medicant can be administered through the catheter. The control tap can be moved to a third alternative position where only the blood supply line and the medicant supply are connected. This position allows for example aspiration of blood from the blood supply line into a syringe attached to the third flow line. The control tap can be moved to a closed position in which all of the flow lines are closed and no blood can flow through the control tap, and no medicant can be delivered through the control tap.

It is necessary to ensure that the patient is not over-transfused, i.e. to ensure that the total volume of blood transfused to the patient, and also the rate of blood flow transfused to the patient, are not too great for the patient to accommodate. The chances of over-transfusion increase significantly with smaller patients having a smaller circulating blood volume. Over transfusion can have serious consequences, such as heart failure, pulmonary edema or death.

With babies and small children in particular (for example weighing up to around 10 kg), the known gravity flow and pressurised transfusion arrangements are not suitable because the volume of blood, and the rate of flow of blood, transfused to the patient cannot be sufficiently well controlled to avoid the possibility of over-transfusion.

One attempt to avoid the over-transfusion of a small patient is to provide smaller blood bags, in particular having a volume of 50 ml. However, the provision of smaller blood bags is not often utilised as they are impractical for all but the smallest patients, and since the volume of blood in storage may not always be sufficient to meet a large demand hospitals typically prefer to store the blood in larger bags which can be used for all patients.

When providing a transfusion to a small patient, therefore, the medical professional will often have to use a blood bag of 350 ml, in the knowledge that the patient can accommodate only a small proportion of that blood during the transfusion procedure. The gravity feed and pressurised arrangements described above are therefore typically not used for smaller patients, and instead the medical professional will take much greater control of the volume and rate of blood being transfused. The method most widely practised is to connect the control tap to the blood supply line and catheter supply line as usual, and to connect a syringe to the third flow line. The control tap is operated so that the blood bag is not connected directly to the patient, and instead the control tap is moved so that the syringe is alternately connected to the blood bag and to the patient. The control tap is therefore not moved to the fully-open position nor the first position as described above, but is instead alternated between the second alternative positions and the third alternative position.

When the syringe is connected to the blood bag the medical professional draws blood into the syringe, and when the syringe is connected to the patient the medical professional expels blood from the syringe to the patient. In this way, a controlled volume of blood can be transfused to the patient, at a controlled rate, directly by the syringe.

This widely-practised method is not foolproof, however, and the over-transfusion of a small patient can still occur. Thus, when the blood transfusion has been completed, or has been temporarily suspended whilst other medical procedures are undertaken, it is necessary to ensure that the blood flow along the catheter line is stopped. Human error can occur, however, and instead of moving the control tap to the closed position, it can inadvertently be moved to the fully-open position or to the first alternative position in which positions the blood bag is connected directly to the patient.

In a conventional control tap only a single (closed) position is available in which there is no blood flow through the control tap, whereas there are four open positions in which blood can flow through the control tap. In three of the open positions blood can flow to the patient. Extreme care must therefore be exercised by all personnel having access to the control tap to ensure that the control tap is closed when the transfusion has been completed or temporarily suspended.

The manufacturers of control taps seek to reduce the likelihood of human error occurring by providing visual indicators on the control tap to show which of the flow lines are interconnected or open. A typical visual indicator is to provide the manually-operable control lever with arms which project in the same direction as the open conduits within the tap, so that the orientation of the arms of the control lever relative to the flow lines of the control tap enable the user to determine the operational position of the control tap. In addition, many types of control tap are fitted with detents so that the user feels a variation in resistance as the control tap is moved between its different positions, the control tap "clicking" into place in each of its operational positions, including its closed position. The provision of detents helps the user to ensure that the control tap has been correctly moved to a desired position, and reduces the likelihood that the control tap will be inadvertently moved from its desired position.

As an additional precaution, some medical professionals fit a series of control taps into the supply line between the blood bag and the catheter when transfusing a small patient. By arranging a series of control taps, it is intended that the chance of human error in leaving all of the taps in their incorrect position (or alternatively stated of not placing at least one of the taps into a closed position when the transfusion is stopped or temporarily suspended) is reduced. Once again, however, whilst this precaution reduces the likelihood of human error it does not avoid it altogether.

None of the visual indicators, the provision of detents, nor the precaution of using a series of taps, therefore avoids the possibility of human error. The doctors and other medical professionals who provide transfusions in emergency situations are all highly trained, but emergencies are inevitably highly stressful situations, where the medical professional often has to address several problems in parallel. The incidence of human errors in these particular situations is understood to be high and the impact of an over-transfusion on a small patient is so significant that there is a need to reduce or avoid the likelihood of human error in these circumstances.

### SUMMARY OF THE INVENTION

The inventor has appreciated the possibility of human error occurring during transfusion, and has sought to reduce or avoid the likelihood of such human error.

According to the present invention, there is provided a transfusion control tap having three flow lines and a rotatable control member, the rotatable control member having conduits or lumens which can be aligned with chosen flow lines to interconnect those flow lines, characterised in that the control member has only two lumens whereby only two of the flow lines can be interconnected at a time.

Preferably, the flow lines are arranged in the form of a "T". The angles between each pair of adjacent flow lines therefore differ, so that it is not possible to interconnect each of the flow lines with both of its neighbours.

Accordingly, the control tap does not have a "fully open" position corresponding to that of a conventional control tap as described above. Also, the control tap is designed to be connected to the blood bag supply line and catheter line so that it does not have the "first alternative position" as described above. Thus, the control tap can be configured so that the blood supply line cannot be connected to the catheter line. When in use for the transfusion of a small child, the control tap can be moved between positions corresponding to the second and third alternative positions described above, i.e. with the blood supply line and the catheter line alternately connected to the third (syringe) flow line.

Preferably, the control tap has a control lever having two arms corresponding in position to the two lumens of the control member, so as to provide a visual indication of the operating position of the control tap.

Desirably, the control tap has a detent position corresponding to each of its operating positions. Preferably, the control tap has a further detent position corresponding to the fully closed position, i.e. the position in which none of the flow lines are open.

Preferably, two of the flow lines are aligned, and the third flow line is perpendicular to said two of the flow lines. In such a configuration the flow lines are in a right-angled "T" configuration similar to that of a conventional control tap. Such embodiments will require the least amount of re-tooling by the control tap manufacturer as the body of the control tap can match a conventional control tap, with only the rotatable control member (and the control lever if that includes a visual indicator of operating positions) needing new or modified tooling.

### BRIEF DESCRIPTION OF THE PREFERRED EMBODIMENTS

The invention will now be described in more detail, by way of example, with reference to the accompanying schematic drawings, in which:
- Fig.1: shows a control tap according to the present invention; and
- Fig.2: shows a blood transfusion arrangement using the invented control tap.

### DETAILED DESCRIPTION

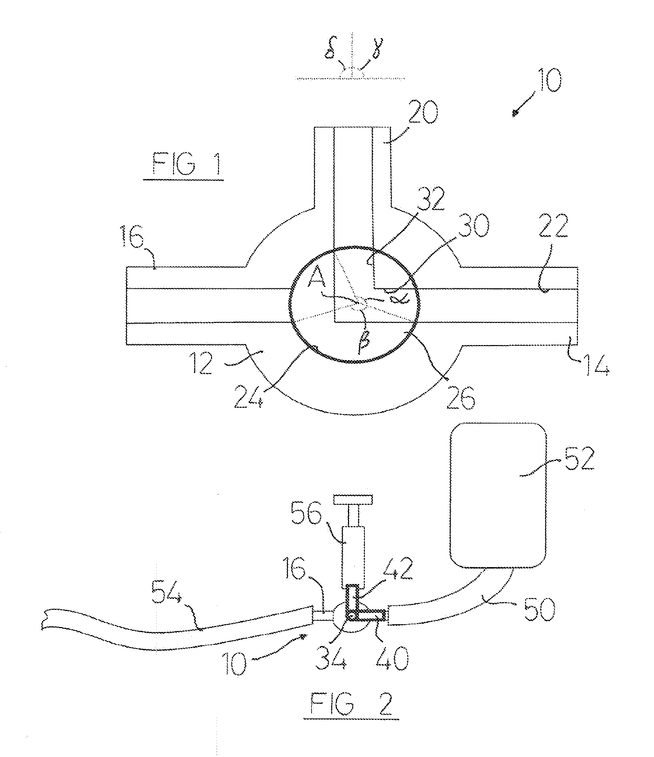

The control tap 10 shown in Figs.1 and 2 shares many features of a conventional control tap. The features which are shared with a conventional control tap will be described first for ease of reference.

The control tap 10 has a substantially rigid body 12 having three flow lines 14, 16 and 20. Each of the flow lines 14, 16 and 20 comprises a hollow tube with an internal conduit or lumen 22 of substantially circular cross-section, and an outer substantially circular periphery (in cross-section). Though not shown in the figures, in practice each flow line 14, 16, 20 will carry a connector by which it may be connected to a flexible tube or to a syringe (as described in relation to Fig.2 below). The connector may be a screw connector if it is desired to provide a secure connection to the flexible tube or syringe.

The body 12 of the control tap 10 has circular bore 24 which accommodates a cylindrical control member 26, the control member 26 being rotatable within the bore 24. The control member 26 has interconnected lumens 30, 32 and rotation of the control member can interconnect respective flow lines 14, 16, 20 by way of the lumens 30, 32.

The control member has a control lever 34 (Fig.2) which can be rotated by a user whereby to rotate the control member 26 within the bore 24. The control lever 34 has an arm 40 aligned with the lumen 30 and an arm 42 aligned with the lumen 32, so that the control lever 34 provides a visual indication of the operating position of the control tap 10.

The control tap 10 differs from the conventional control tap in having only two lumens 30, 34, i.e. a conventional control tap has another lumen aligned with the lumen 30, a conventional control tap therefore having a lumen which can be aligned with each of the flow lines 14, 16, 20.

The control member 26 is rotatable around a rotation axis A. The control member can be freely rotatable, or preferably has one or more detent positions providing a variation in the resistance to rotational movement, so that the control member 26 can readily be moved between its operating positions. In one embodiment including detents, the outer periphery of the control member 26 carries one or more raised lugs and the inner surface of the bore 24 has one or more cooperating recesses, the lug(s) locating in the recess(es) in the various operating positions.

In use as shown in Fig.2, the flow line 14 is connected to a length of flexible tubing 50, the other end of which is connected to a blood bag 52. The length of flexible tube 50 therefore provides the blood supply line. The flow line 16 is connected to another length of flexible tubing 54, the other end of which is connected to a catheter inserted into a patient (not shown). The flow line 20 is connected to a syringe 56. With the control tap 10 in the operating position shown in Fig.2 (which corresponds to the third alternative position of a conventional control tap described above) the lumens 30 and 32 interconnect the flow lines 14 and 20, and therefore interconnect the syringe 56 and the blood bag 52. In this operating position, a medical professional can draw a desired volume of blood from the blood bag 52 into the syringe 56.

It will be understood that in such an arrangement it is not necessary to suspend the blood bag 52 above the height of the patient or the syringe, and the blood bag merely acts as a reservoir for the blood which is to be delivered to the patient. It is nevertheless expected that for hygiene and convenience reasons the blood bag 52 will often be suspended on a transfusion stand as in a gravity-fed transfusion arrangement.

When the medical professional has drawn a chosen volume of blood into the syringe 56, the control lever 34 is rotated through 90° anticlockwise as drawn so that the lumen 30 is aligned with the flow line 20 and the lumen 32 is aligned with the flow line 16. The lumens 30, 32 therefore interconnect the syringe 56 and the catheter line 54, and the medical profesional can administer a controlled volume of blood into the patient, at a controlled rate.

The process can be repeated as often as is required, and when it is desired to stop the transfusion, or temporarily to suspend the transfusion whilst other medical procedures are undertaken, the control tap is moved to a closed position in which one or both of the lumens 30 and 32 are moved out of alignment with any of the flow lines 14, 16 and 20. It will be understood that rotating the control member 24 clockwise through 90° or through 180° from the position of Fig.1 will result in the control tap being "closed" since only one of the lumens 30 or 32 will be aligned with a supply line. Also, rotating the control member 24 clockwise from the position of Fig.1 through 45°, 135°, 225° or 315° will also result in the control tap being closed since in those positions neither of the lumens 30 or 32 will be aligned with a flow line. The control tap 10 can have a detent position corresponding to any or all of these closed positions so as to define a closed position for the control tap 10.

As will be understood from the foregoing description, the control tap 10 is intended to remove the possibility that the flow line 14 can be connected directly to the flow line 16, so that the medical professional can ensure that blood cannot flow directly from the supply line 50 to the catheter line 54, regardless of human error. In order to ensure that no direct connection can be made between the flow lines 14 and 16 the angle α shown in Fig.1 must be smaller than the angle β.

The angle α is the maximum angle between the lumens 30 and 32 which can provide a continuous flow path through those lumens. The angle β is the minimum angle between the conduits or lumens 22 within the respective flow lines 14 and 16. The angle α will increase as the diameter of the lumens 30, 32 increases relative to the diameter of the control member 26. The angle β will reduce as the diameter of the lumens 22 increase relative to the diameter of the control member 26. The designer of the control tap 10 can determine the relative diameters of the lumens 20, 30, 32 and the control member 26 in order to ensure that the angle α will be smaller than the angle β, so that the control tap cannot be moved to a position in which the lumens 30, 32 provide a continuous flow path between the flow line 14 and the flow line 16.

It will be understood that the precise "T" shape of the control tap 10 is not required for the invention to operate, i.e. it is not necessary that the flow line 14 be aligned with the flow line 16 and for the flow line 20 to be perpendicular to both of the flow lines 14 and 16. It is of course desired that the angle γ between the flow line 14 and the flow line 20 exactly matches the angle δ between the flow line 16 and the flow line 20, this being the same as the angle between the lumens 30 and 32. In the perpendicular arrangement of the control tap 10 the angles γ and δ are 90° but in alternative embodiments these angles could be less than 90°, for example 60°. It is also possible that the angle γ/δ could be greater than 90°, but that is not preferred as angles approaching 120° would not enable the control tap to prevent direct flow from the supply line to the catheter line.

## Claims

1. A transfusion control tap (10) having three flow lines (14, 16, 20) and a rotatable control member (26), the rotatable control member having lumens (30, 32) which can be aligned with respective flow lines to interconnect those flow lines, **characterised in that** the control member has only two lumens whereby only two of the flow lines (14, 16, 20) can be interconnected at a time.

2. A transfusion control tap (10) according to claim 1 in which the rotatable control member (26) can interconnect the first flow line (14) and the third flow line (20), and can interconnect the second flow line (16) and the third flow line (20), but cannot interconnect the first flow line (14) and the second flow line (16).

3. A transfusion control tap (10) according to claim 1 or claim 2 in which the angle (γ) between the first flow line (14) and the third flow line (20), the angle (δ) between the second flow line (16) and the third flow line (20), and the angle (360°-(γ+δ)) between the first flow line (14) and the second flow line (16), are not all the same.

4. A transfusion control tap (10) according to any one of claims 1- 3 in which the angle (γ) between the first flow line (14) and the third flow line (20), and the angle (δ) between the second flow line (16) and the third flow line (20) are substantially identical, and differ from the angle (360-(γ+δ)) between the first flow line (14) and the second flow line (16).

5. A transfusion control tap (10) according to claim 4 in which, when measured from the axis of rotation (A) of the rotatable control member (26), there is a maximum angle (α) between the lumens (30, 32) which can provide a continuous flow path through the lumens, in which there is a minimum angle (β) between the first flow line (14) and the second flow line (16) which can provide a continuous flow path between those flow lines, and in which the maximum angle (α) between the lumens (30, 32) is less than the minimum angle (β) between the first flow line (14) and the second flow line (16).

6. A transfusion control tap (10) according to any one of claims 1-5 in which the flow lines (14, 16, 20) are arranged in the form of a "T".

7. A transfusion control tap (10) according to claim 6 in which the first and second flow lines (14, 16) providing the top bar of the "T" are substantially aligned, and are each substantially perpendicular to the third flow line (20) providing the leg of the "T".

8. A transfusion control tap (10) according to any one of claims 1-7 in which the angle (γ) between the first flow line (14) and the third flow line (20), and the angle (5) between a second flow line (16) and the third flow line (20) are both approximately 90°, so that the angle (360°-(γ+δ) between the first flow line (14) and the second flow line (16) is approximately 180°.

9. A transfusion control tap (10) according to any one of claims 1-8 having a control lever (34) with two arms (40, 42), the arms corresponding in position to the two lumens (30, 32).

10. A transfusion control tap (10) according to any one of claims 1-9 having a detent position corresponding to each of its operating positions.

11. A transfusion control tap (10) according to claim 10 having a further detent position corresponding to a fully closed position.
